(19) **Europäisches Patentamt / European Patent Office / Office européen des brevets**

(11) **EP 3 093 023 B1**

(12) # EUROPEAN PATENT SPECIFICATION

(45) Date of publication and mention
of the grant of the patent:
**04.11.2020 Bulletin 2020/45**

(21) Application number: **15740157.1**

(22) Date of filing: **23.01.2015**

(51) Int Cl.:
**C12N 5/074** (2010.01)    **A61K 35/32** (2015.01)

(86) International application number:
**PCT/JP2015/051887**

(87) International publication number:
**WO 2015/111712 (30.07.2015 Gazette 2015/30)**

(54) **PHARMACEUTICAL COMPOSITION FOR CANCER TREATMENT**

PHARMAZEUTISCHE ZUSAMMENSETZUNG ZUR KREBSBEHANDLUNG

COMPOSITION PHARMACEUTIQUE POUR LE TRAITEMENT DU CANCER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.01.2014 JP 2014011729**

(43) Date of publication of application:
**16.11.2016 Bulletin 2016/46**

(73) Proprietor: **QUARRYMEN & CO. INC.**
**Tokyo 157-0066 (JP)**

(72) Inventor: **UEDA, Minoru**
**Kanagawa 240-0105 (JP)**

(74) Representative: **Prüfer & Partner mbB**
**Patentanwälte · Rechtsanwälte**
**Sohnckestraße 12**
**81479 München (DE)**

(56) References cited:
**EP-A1- 2 307 539     EP-A1- 2 554 175
EP-A1- 2 832 851     WO-A1-2013/147082
JP-A- 2011 529 329     JP-A- 2013 018 756**

- **ARANHA A M F ET AL: "Hypoxia Enhances the Angiogenic Potential of Human Dental Pulp Cells", JOURNAL OF ENDODONTICS, LIPPINCOTT WILLIAMS & WILKINS, PHILADELPHIA, PA, US, vol. 36, no. 10, 1 October 2010 (2010-10-01), pages 1633-1637, XP027290274, ISSN: 0099-2399 [retrieved on 2010-07-15]**
- **LI ET AL: "Effects of hypoxia on proliferation and differentiation of myoblasts", MEDICAL HYPOTHESES, EDEN PRESS, PENRITH, US, vol. 69, no. 3, 13 July 2007 (2007-07-13), pages 629-636, XP022140498, ISSN: 0306-9877**
- **YING WANG ET AL: "The hypoxia-inducible factor [alpha] pathway couples angiogenesis to osteogenesis during skeletal development", JOURNAL OF CLINICAL INVESTIGATION, vol. 117, no. 6, 1 June 2007 (2007-06-01), pages 1616-1626, XP055401850, US ISSN: 0021-9738, DOI: 10.1172/JCI31581**
- **MASAHITO FUJIO ET AL: "Conditioned media from hypoxic-cultured human dental pulp cells promotes bone healing during distraction osteogenesis : Hypoxic culture condition increases the angiogenic factors from hDPCs", JOURNAL OF TISSUE ENGINEERING AND REGENERATIVE MEDICINE, vol. 11, no. 7, 1 July 2017 (2017-07-01), pages 2116-2126, XP055401803, US ISSN: 1932-6254, DOI: 10.1002/term.2109**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(Cont. next page)

• **TOMOKAZU HASEGAWA: 'Investigation of New Strategy for Periodontal Tissue Regeneration Using with Human Immortalized Periodontal Ligament Cell Line Derived from Deciduous Teeth' THE JAPANESE JOURNAL OF PEDODONTICS vol. 50, no. 1, 2012, pages 7 - 14, XP055357362**

## Description

### Technical field

The present invention relates to a pharmaceutical composition for cancer treatment and pharmaceutical preparation for cancer treatment thereof as active ingredient. Specifically, it relates to the pharmaceutical composition for cancer treatment by using a culture supernatant derived from mammal dental pulp stem cell and the pharmaceutical preparation comprising thereof as an active ingredient.

### Background Art

It is known that the hypoxic response in microenvironment in a body affects organ formation during development, proliferation of stem cell, and the like. Also, it is known that the effect of the hypoxic response relates to disease such as cancer and ischemic disorder.

Also, the hypoxic environment regulates expression of a variety of genes and controls cell response such as cell growth, differentiation, apoptosis and the like.

On the other hand, a variety of anticancer agents have been developed for cancer therapy. For example, there are mentioned as anticancer agents: cytarabine, fluorouracil, mercaptopurine and thioguanine as DNA synthesis inhibitors; vinblastine, vincristine and procarbazine as vinca alkaloids; mustine, cyclophosphamide and cisplatin of alkylating agent; actinomycin D, doxorubicin, mitomycin, mitramycin and bleomycin as antibiotics; and glucocorticoid, estrogen, anti-estrogen and androgen as steroid hormones and the like.

Cytarabine inhibits DNA polymerase, and fluorouracil inhibits thymidylate synthase to suppress pyrimidine synthesis. Mercaptopurine or thioguanine inhibits purine synthesis. Vinblastine or vincristine acts specifically to DNA in M phase, and destroy the spindle by binding to tubulin to stop mitosis of a cell. Procarbazine causes depolymerization of double strand DNA to inhibit DNA synthesis, and mustine, cyclophosphamide, cisplatin and the like cause covalent cross-link to inhibit DNA synthesis. Actinomycin D, doxorubicin, mitomycin, mitramycin and the like are intercalator, which are intercalating between a space formed by base pairs in double strand DNA, and block RNA synthesis. Also, bleomycin causes the breakage of the double strand DNA. Glucocorticoid, estrogen, anti-estrogen, androgen and the like inhibit the protein synthesis after RNA synthesis.

Other than that, a lot of anticancer drugs have been developed and used for treatment to exert a certain effect of treatment.

### Prior Art

[Patent Document]

[Patent Document 1] WO 2011/118795
[Patent Document 2] WO 2013/147082
[Patent Document 3] EP 2 307 539

[Non Patent Document]

[Non Patent Document 1] Yue Wang, et al., PLOS ONE, January 2013, Vol. 8, Issue 1, e54296
[Non Patent Document 2] M. Celeste Simon and Brian Keith, Nature Reviews/ Molecular Cell Biology, April 2008, Vol. 9, pp. 285-296
[Non Patent Document 3] Aranha A M F, et al., Journal of endodontics, October 2010, Vol. 36, no. 10: 1633-1637

### Summary of the Invention

### Problems to be solved by the Invention

WO 2011/118795 discloses that composition for treatment of damaged part of target tissue containing culture supernatant of stem cell obtained by culturing stem cell (hereinafter, it is referred to as, "prior art 1").

The prior art 1 is an excellent art from the view point that it found that culture supernatant of stem cell, which is cultured under the condition of standard oxygen concentration and contains at least two cytokines selected from the group consisting of vascular endothelial cell growth factor (VEGF), hepatocyte growth factor (HGF), insulin like growth factor (IGF), platelet derived growth factor (PDGF) and insulin like growth factor (IGF-β), may be used for treatment for damaged tissue by a variety of disease; for example, central nervous tissue, dermal tissue, periodontal tissue, bone tissue, brain tissue and retinal tissue; and it developed the drug for damaged tissue by cardiac disease or cerebral vascular disease, those are one of the major causes of death. However, the prior art does not consider these agents for tumor (cancer), which is also one of the major cause of death.

WO 2013/147082 describes the preparation of immortalized stem cells from dental pulp stem cells. The cells secrete VEGF and IGF. EP 2 307 539 describes the use of hypoxic conditions to increase the efficiency of establishing IPs cells. Aranha A M F, et al., Journal of endodontics, October 2010, Vol. 36, no. 10: 1633-1637, describes the use of low oxygen levels to simulate the conditions of dental pulp hypoxia developed by the rupture of the neuro-vascular bundle in intrusive laxations and evulsions. It teaches that hypoxia induces complex and cell type-specific pro-angiogenic responses and suggests that VEGF participates in the revascularization of hypoxic dental pulps.

As to the treatment for cancer, chemotherapy is usually conducted using the anticancer agent; however, they often become ineffective (resistant) in clinical. The mechanisms of the resistance are, for example, depending on the biological reaction derived from the cancer patient such as inactivated anticancer agent or enhanced metabolism in their livers; or on the biochemical change of cancer cells in a cellular level. In the latter, it is considered that following issues related to multi-drug resistance were occurred: changed membrane transport mechanism of anticancer agent, amplified target enzyme or protein, decreasing of drug activation mechanism or enzyme activity, for example, DNA repair mechanism, inactivation mechanism of anticancer agent, and the like. Furthermore, many anticancer drugs have strong side effects that cause the death of the patient.

Therefore, there are strong needs for a method for cancer treatment having few side effects. Immunotherapy stands out as the method for cancer treatment having few side effects; however, the method has low capability for controlling tumor so that it is difficult to certainly eliminate them. This is derived from the immunotherapy utilizing lymphocyte or NK cell. These cells are mainly responsible for the immunity, and are originally responsible for infectious disease so that they have slight inhibitory effects on solid tumor.

Therefore, there are strong social requests to develop a novel cancer treatment agent, which is highly effective on the solid tumor without side effects such as those given by chemotherapeutic agents, and hardly causes drug-resistance.

**Means for solving the problem**

The invention is defined by the appended claims. The inventors conducted a research of biological ability of dens deciduous stem cell (SHED) under the circumstance, and found that the culture supernatant of SHED (hereinafter, it is sometimes referred to as "SHED-CM") controls the function of macrophage (hereinafter, it is sometimes referred to as, "M"), and complete the invention.

That is, the first aspect of the invention is a pharmaceutical composition for cancer treatment prepared by a method comprising; manufacturing immortalized stem cells of dens deciduous dental pulp stem cells derived from mammal dental pulp by transfection; and preparing a conditioned medium by culturing said immortalized stem cells in a serum free medium for a predetermined period under the condition of hypoxic concentration of 0.5% or more but 10% or less of oxygen concentration between the temperature of 23 to 27 °C.

Here, it is preferable that the predetermined time is 40 to 56 hours, and that the hypoxic concentration is 5% or less of oxygen concentration. Also, it is preferable that said hypoxic concentration is 1% or less of oxygen concentration. It is preferable that the immortalized stem cells are manufactured by transfecting 4 genes selected from the group consisting of hTERT, bmi-1, E6, E7, Oct3/4, Sox2, Klf4, c-Myc, and p16INK4a into the dens deciduous dental pulp stem cells.

It is preferable that the pharmaceutical composition for cancer treatment prepared by the above method further contains more than five times of transforming growth factor β (TGF-β1) compared to the condition medium prepared by culturing the cell on the same condition except 20% of oxygen concentration. It is preferable that the pharmaceutical composition for cancer treatment further contains more than three times of stromal cell-derived factor (SDF-1).

Also, another aspect of the invention is the pharmaceutical preparation for cancer treatment that contains the pharmaceutical composition as active ingredient. It is preferable that the cancer is the solid tumor.

**Advantageous Effect of the Invention**

According to the present invention, a pharmaceutical composition is obtained by manufacturing immortalized stem cells of dens deciduous dental pulp stem cells derived from mammal dental pulp by transfection; and preparing a conditioned medium by culturing said immortalized stem cells in a serum free medium for a predetermined period under the condition of hypoxic concentration of 0.5% or more but 10% or less of oxygen concentration between the temperature of 23 to 27 °C; wherein, the composition contains significantly higher concentration of IGF-1 and VEGF compared to those cultured at 20% of oxygen concentration.

By administrating the pharmaceutical composition, population of macrophage accumulating around solid tumor cells is controlled to control the growth of the cancer cell or kill the tumor cells.

**Brief Description of Drawings**

Fig. 1 is a graph showing a relationship between culturing period and population doubling times of the immortalized stem cells and non-immortalized stem cells. In Fig. 1, SHED-T shows the immortalized stem cells, and SHED-C shows the non-immortalized stem cells.

Fig. 2 is the graph showing the results of STRO-1 expression in both SHED-C and SHED-T (see, Figs. 2(A) to (D)). In the figure, PD20 means that the population doubling time is 20; PD30 means that it is 30, and PD40 means that it is 40.

Fig. 3 shows the tissue staining images of SHED-C (D) to (F) and SHED-T (A) to (C) at each time point of the individual population doubling time shown in the following Fig. 4.

Fig. 4 is the graph showing the relationship between the population doubling time (number of times) and mass of newly generated bone. In the figure, ** shows $p < 0.05$, *** shows $p < 0.01$. The mass of newly generated bone are calculated by using the following equation.

$$\text{The mass of newly generated bone} = \text{area of the newly generated bone} / \text{sight area} \times 100$$

Fig. 5 shows the result of time course change of tumor volume after injecting SCCVII under the skin when the culture supernatant of SHED cultured under different condition is administered. In the figure, * shows $p < 0.05$, ** shows $p < 0.001$ obtained from ANOVA analysis.

Fig. 6 shows the tumor (A) having the volume over 10 mm in diameter, and complete regression thereof (B).

Fig. 7 shows the time-dependent survival ratio of each group of mice, which were injected SCCVII subcutaneously.

Fig. 8 is the in vivo image of the mouse bearing the solid tumor, to which $1 \times 10^7$ cells of macrophage labeled with IVIS (registered trade mark) XenoLight DiR (Summit Pharmaceutical International Co.) were injected through its tail vein.

Fig. 9 shows the population change of intraperitoneal macrophage in the mouse bearing the solid tumor formed by SCCVII.

Fig. 10 shows histological autopsy (hematoxylin-eosin staining image) of the solid tumor formed by mouse squamous cell carcinoma, SCCVII, is subcutaneously injected.

**Mode for Carrying Out the Invention**

The present invention is explained in detail in below.

In order to obtain the immortalized stem cell that is used in a method of preparing the pharmaceutical composition of the present invention, firstly, the stem cell is isolated from a mammal mesenchymal cell, early generated embryo, and somatic cells. As the mammal animal, it is preferable to be selected from the group consisting of human, swine, equine, and monkey, because the cells obtained from the mammal are genetically similar to the human cells and are not so dangerous for infectious disease.

In the present specification, the term, "mesenchymal cell", is defined as the cells having differentiation ability into the cells belonging to the mesenchymal such as osteoblast, adipocyte, muscle cell, cartilage cell, and the like. As the mesenchymal cell, there are mentioned such as the dental pulp cell, bone marrow cell, umbilical cell and adipocyte of the animals as mentioned above. Also, the term, "early-generated embryo", is defined as the embryo in the early stage by the blastocyst, namely, it is in the progressed stage than the fertilized egg, and necessary for establishing ES cell. The term, "somatic cell", is defined as the general term of the cell except a germ cell among those which compose of a living body.

Furthermore, the term, "dental pulp cell", is a kind of stem cells included in the nerve of the teeth, which has the regeneration ability. It has properties such that it is protected by hard material, teeth, which does not permeate UV light or radioactive ray, so that genes in them are hardly damaged. The term, "bone marrow cell", is defined as the general name of the cells obtained from bone-marrow aspirate, and it includes a leukoblast cells such as myeloblast cells, erythroblast cells, bone marrow megakaryocyte cells, plasma cells and the like.

In the present specification, the term, "umbilical cell", is that exists in the umbilical cord, which binds the embryo and a placenta. It is contains umbilical cell-rich umbilical blood in the umbilical cord.

As the genes introduced into the stem cells as described above, hTERT, bmi-1, E6, E7, Oct3/4, Sox2, Klf4, c-Myc, p16INK4a, and the like are mentioned. hTERT is a gene for telomere repair enzyme; bmi-1 is the gene of Bmi-1, which is one of proteins composing of polycomb group complex. Here, Bmi-1 is necessary for maintaining the hematopoietic stem cells, which has functions that they increase the stem cell numbers through their activity enhancement.

Both E6 and E7 are early genes of either HPV-16 or HPV-18. Also, Oct3/4 is the gene that cooperates with Sox2 to activate the transcription of the target gene. Klf4 (Kruppel type transcription factor 4) regulates the genes relating to the cell division and the embryogenesis, and it relates to the gastrointestinal system cancer as the tumor suppressor.

Sox2 belongs to SRY-related HMG box gene family, and it is known as the gene that relates to the maintenance of undifferentiated functions (totipotency). c-Myc is a cancer promoting gene, and it promotes both of survival and death of the cell in the c-Myc-induced tumor. p16INK4a is the gene which plays an important role to control the cell cycle of the tumor cell.

The manufacturing of immortalized stem cell is explained as an example by using the dental pulp cells obtained from the human exfoliated dens deciduous teeth in below.

Firstly, the exfoliated dens deciduous are disinfected by using a disinfection agent, for example, chlorhexidine, Isodine, and the like. After that, a crown of the tooth is divided, and dental pulp is collected by using dental reamer.

Obtained dental pulp tissue is suspended in the basal media, Dulbecco's modified eagle's MEM (Dulbecco's Modified Eagle's Medium, herein below, it is sometimes referred to as "DMEM") containing, for example, 5 to 15% (v/v) of calf serum (herein below, it is sometimes referred to as "CS"), and 50 to 150 U/mL of antibiotics. Then, they are treated with both of 1 to 5 mg/mL of collagenase and 1 to 5 mg/mL of dispase at 37 °C for 0.5 to 2 hours.

As the basal media, other than DMEM, Iscove's Modifed Dulbecco's Medium (IMDM) (GIBCO, etc.), Ham's F12 medium (HamF12) (SIGMA, GIBCO, etc.), RPMI1640 medium, and the like may be used. Also, a mixed media comprising at least two media may be used. As the example of the mixed medium, a medium including IMDM and HamF12 in equal amount (for example, it is commercially available as the product name: IMDM/HamF12 (GIBCO)) is mentioned.

As the components to be added to the media, there are mentioned, for example, serum such as fetal calf serum (hereinbelow, it is sometimes referred to as "FCS"), human serum, sheep serum and other serum, serum replacement (Knockout serum replacement (KSR), etc.), bovine serum albumin (it is sometimes referred to as "BSA"); antibiotics such as penicillin, streptomycin and others, various vitamins, various minerals, and the like.

The basal medium may be also used to culture for the selection of cells as mentioned below, and used to culture for the selected cells.

After the dental pulp tissue in the suspension is treated with the enzyme, the suspension is centrifuged for 3 to 10 minutes (3,000 to 7,000 rpm) to collect the dental pulp cells. Depending on the necessity, the cells are selected by using a cell strainer. The selected cells are, for example, resuspended in 3 to 6 mL of the basal medium, and are plated in dishes having 4 to 8 cm of diameter for adherent cell culture.

Subsequently, the medium, for example, DMEM containing 10 % FCS, is added to the dishes, and then the cells are incubated in 5% $CO_2$ incubator at 37 °C for about 2 weeks. The medium are removed from the dishes, and then the cells are washed with PBS and the like for 1 to several times. Instead of the medium removal and washing of the cells, the adherent dental pulp stem cells which formed colonies may be collected. The adherent dental pulp stem cells are treated with a solution including both of 0.025 to 0.1 % trypsin and 0.3 to 1 mM EDTA for several minutes at 37 °C to be detached from the dish. Next, the detached cells are collected.

Subsequently, the selected adherent cells as described above are cultured. For example, the dental pulp stem cells obtained as mentioned above are plated into the dishes for the adherent cell culture, and then cultured under the conditions of 5% $CO_2$ and at 37 °C in the incubator.

For passage culture, the cells are collected by using trypsin and EDTA as mentioned above when the cells become sub-confluent or confluent with macroscopic observation. Then, the cells are plated again in the culture dish containing the fresh culture medium.

Here, the term, "sub-confluent", means that the bottom surface of the culture vessel where the cells adhere is covered by the cells about 70 % thereof. For example, from 1 to 8 times of the cell passage are conducted to select the cells, and then the selected cells are allowed to grow to reach the necessary cell number, for example, about $1 \times 10^7$ cells/mL. After culturing the cells as described above, the cells are collected and stored in liquid nitrogen. The cells collected from a variety of donors may be stored in the form of dental pulp stem cell bank.

Next, primary-cultured cells are obtained from primary cultured the stem cells, to which the 4 genes are introduced to manufacture gene-transduced cells. The genes transduced here are preferably 4 genes selected from the group consisting of hTERT, bmi-1, E6, E7, Oct3/4, Sox2, Klf4, c-Myc, and p16INK4a. The transfected genes, hTERT, bmi-1, E6, and E7, into the primary-cultured cells give the immortalized cells having higher population doubling time. Here, hTERT is the gene for human telomerase reverse transcriptase; bmi-1 is the polycomb group gene relating to auto-reproduction or differentiation regulation of the stem cell. E6 and E7 are genes existing in an open reading frame coding early gene used to replicate human papilloma virus itself.

Such genes may be introduced as follows.

A plasmid for incorporating the target genes is prepared, and then it is inserted into a shuttle vector, for example, pShuttle2 to clone the genes. By using the shuttle vector, E. coli is transformed to select kanamycin resistant transformants. Plasmid DNAs of the selected kanamycin resistant transformants are purified for analyzing restriction sites thereof. Thus, the transformants are identified.

Next, an expression cassette is cut out from the shuttle vector by using restriction enzymes, for example, PI-Sce I and I-Cue I, and then it is ligated into adenovirus vector, for example, Adeno-X viral DNA. Obtained ligation product is cleaved by using Swa I, and it is used to transform the E. coli.

From the obtained transformants, ampicillin resistant ones are selected. The recombinant adenovirus DNA with inserted genes is purified for analyzing the restriction sites thereof. Thus, the transformants are identified..

Next, the adenovirus is digested by using Pac I, and then it is transfected into HEK 293 cells. The recombinant adenovirus is propagated, and then collected to determine their titers. The virus is purified according to a conventional method, and it is passed to the target cell, SHED.

Cell population of viral infection is stained by using FITC according to the conventional method, and then STRO-1 positive cells among them are detected by using a flow cytometer. Here, STRO-1 is considered as one of markers for the mesenchymal stem cell having pluripotency in the bone marrow, and it is considered to become an index for cell immortalization.

According to the above-mentioned procedure, the immortalized stem cell derived from the dental pulp may be obtained.

Next, the obtained immortalized stem cell is cultured in the basal medium, for example, DMEM supplemented with 10% FBS under the condition of 5% $CO_2$ at 37 °C for 24 to 48 hours to obtain the culture supernatant. In order to collect the culture supernatant, for example, a Komagome type pipette and the like may be used. The collected culture supernatant may be used as an active ingredient for the pharmaceutical composition of the present invention as it is. Also, it may be used as the active ingredient after treatments such as condensation, replacement of the solvent, dialysis, lyophilization, dilution and others.

As described below, the culture supernatant of the immortalized stem cell obtained as mentioned above includes a variety of growth factors, and it shows many functions without higher purification. Namely, by using the culture supernatant, the pharmaceutical composition of the present invention for being employed in many diseases may be produced in a convenient process. Therefore, it prevents decrease of physiological activities of the growth factors caused by the higher purification.

Note that the term, "culture supernatant of the immortalized cell", used in the present invention is defined as the culture supernatant including a variety of biological factors obtained from the immortalized stem cell cultures, and it is the solution that does not include any cells such as the immortalized stem cells and other cells. When the culture supernatant without serum is prepared, it is preferable to use serum-free medium in entire process from initial culture to the passage or at several passage prior to collect the cells.

The dental pulp stem cells, which are selected and cultured by using the above-mentioned method, have the same properties as the primary-cultured cells, firstly plated cells, and obtained from the tissues or cells excised from the living body. In general, the primary cultured cells are important, because they have similar properties to those of the source organ, and they have characteristics close to those of the normal cells. However, it grows slower compared to the lined cell, and sometimes it dedifferentiates during continued culture. Therefore, it is difficult to maintain the primary cultured cell with the original properties.

However, it is preferable for the immortalized stem cells that are used in a method of preparing the pharmaceutical composition of the present invention that they have significantly higher expression ratio of STRO-1, the marker of undifferentiated degree of the cells, compared to that of the non-immortalized dental pulp stem cells at the time point of population doubling time 20 or 40; and show about 1.5 to 3 times higher ratio. Because, the higher expression ratio of STRO-1 becomes the index that the cell has the same properties as that of the primary cultured cell.

Also, the immortalized stem cell that is used in a method of preparing the pharmaceutical composition of the invention secretes at least two growth factors selected from the group consisting of insulin-like growth factor (IGF), vascular endothelial growth factor (VEGF), transforming growth factor-β (TGF-β), and hepatocyte growth factor (HGF) into the culture supernatant. Here, the term, "growth factor", is a general term of polypeptide which promotes the cell division and causes the morphological change or cell hypertrophy. The secreted growth factors are different depending on the cell type, which produces them; and they are roughly classified into epidermal growth factor (EGF), fibroblast growth factor (FGF), nerve growth factor (NGF), tumor growth factor (TGF) and the like.

Furthermore, the receptors on the cell membrane of each cell have tyrosine kinase activity. When the growth factors bind them, tyrosine residues of the proteins are phosphorylated to cause the cell growth or proliferation. It is known that there are several examples that the growth factor becomes a mesoderm inducer in the ontogenesis. Also, it is known that there are several examples that the lymphokine, which modulates the immune system, becomes the mesoderm inducer in the ontogenesis. Such growth factors may be determined by using ELISA assay, microarray assay and the like.

IGF-1 is the polypeptide having highly similar sequence to that of insulin, and it causes reactions such as mitogenesis and the like in the culture cells as insulin does. It is also known that IGF-1 affects the nerve cell growth. Also, VEGF is a member of glycoprotein family involving to formation of blood vessels as vascurogenesis that newly forms them in a region without blood vessel, and angiogenesis that promotes to branch or extend the blood vessel already exists in embryonic stage. TGF-$\beta$ also becomes a powerful growth inhibitor against a variety of the cells, and tightly involves to the cell differentiation, migration or adhesion. TGF-$\beta$ plays important roles in a broad range of areas such as ontogenesis, tissue reconstruction, wound healing, inflammation, immunity, cancer invasion, metastasis, and the like. Furthermore, HGF has a variety of physiological activities involving the regeneration and protection of the tissue and the organ such as the promotion of the cell proliferation, enhancement of cell motion, anti-apoptosis (cell death), morphogenetic induction, angiogenesis and others for the various cells other than hepatocyte.

Each stem cell as mentioned above is cultured, for example, in DMEM supplemented with 15 % FCS at 37 °C in the predetermined term. By this, the protein as mentioned above was obtained in culture supernatant including the growth factors. Note that the culture supernatant of the stem cell includes about 70 types of proteins besides IGF-1, VEGF, TGF-$\beta$, and HGF.

15 mL of the culture supernatant obtained is poured into Amicon Ultra Centrifugal Filter Units-10K (Millipore Limited). Then, it is centrifuged with 4,000 x g for about 60 minutes to concentrate up to about 200 $\mu$L. Next, the same volume of sterilized PBS as the culture supernatant poured into the tube, and it is centrifuged again with 4,000 x g for about 60 minutes to exchange the solvent with PBS. The obtained 200 $\mu$L of the solution is collected into the micro test tube to obtain the condensed stem cell culture supernatant.

Instead of using Amicon as described above, ethanol precipitation may be used for concentration of the culture supernatant. For example, 45 mL of 100 % ethanol is added to 5 mL of the culture supernatant to mix with them, and then stood at -20 °C for 60 minutes. After that, the solution is centrifuged with 15,000 x g for 15 minutes at 4 °C to remove the supernatant.

Next, for example, 10mL of 90 % ethanol is added to the precipitate and mixed well, and then again centrifuged with 15,000 x g for 5 minutes at 4 °C. After removing the supernatant, obtained pellet may be dissolved, for example, in 500 $\mu$L of the sterilized water. After the dissolution, the entire volume of the solution is collected in the micro test tube as the concentrated stem cell culture supernatant.

The obtained culture supernatant as mentioned above may be used as it is, or may be used as diluted one by appropriately dilution with physiologically acceptable solvent such as phosphate buffered saline. Also, the supernatant may be lyophilized according to the conventional method for using as a pharmaceutical composition prepared at the time of use.

The amount of the growth factor in the culture supernatant included in the pharmaceutical preparation is preferably about 50 to 500 weight % against the total dry weight thereof.

As the dosage form of the pharmaceutical preparation, there are mentioned such as powder, liquid, gel, spray, percutaneous absorption system and the like. For example, the pharmaceutical preparation may be prepared by adding additives such as filler, an excipient, an acidity regulator and the like, and may be placed in a small sized container such as a sterile glass ample, serum tube and the like. When using it, the pharmaceutical agent is administrated transnasally or percutaneously at an affected region by using a solution prepared with saline or sterile distilled water for injection. When the pharmaceutical preparation is used for osteogenesis of the alveolar bone or other bones, a scaffolding member such as collagen, $\beta$-TCP and the like may be used, in which those are immersed in the dissolved solution, and they are embedded to the body.

The sub-population of macrophage in the body is controlled by using by a variety method such as intravenous administration of the obtained culture supernatant, thereby treating tumor.

## Example

The present invention is described more specifically by using examples as below; however, it is not limited to the examples.

(Example 1) Preparation of immortalized cell

(1) Construction of vector for plasmid introduction

(1-1) Reagents and the like for plasmid extraction

Kanamycin (Kan), ampicillin (Amp), LB liquid medium and LB agar medium, glycogen, agarose, sterilized water, ammonium acetate, sodium acetate, sodium dodecyl sulfate and RNase A were used. Both 50 mg/mL of kanamycin and ampicillin were prepared to store them as stock solutions at - 20 °C. Glycogen was prepared at the concentration of 20 mg/ml. 10 mg/ml of RNase A was prepared to store at - 20 °C. 10 M (saturated) ammonium acetate ($NH_4OAc$) and 3M sodium acetate (NaOAc; pH 5.2) were prepared.

(1-2) Restriction enzyme and the like

E. coli competent cell (Supercharge EZ10 Electro competent Cells, product code 636756), Swa I (the product code 1111A, Smi I is a comparable one), Xho I (the product code 1094A), T4 DNA Ligase (the product code 2011A), NucleoBond Xtra Midi (the product code 740410.10/.50/.100), NucleoSpin Plasmid (the product code 740588 10/50/250) were purchased from Takara Bio Inc. Pac I was purchased from New England Biolabs.

(1-3) Buffer and the like

1 x TE Buffer (10 mM Tris-HCl (pH 8.0) including ImM EDTA), a mixture of phenol: chloroform: isoamyl alcohol (25:24:1) which is saturated with 100 mM Tris-HCl (pH 8.0), hereinafter, it is referred to as "PCI solution"), was prepared. Either 100 % Ethanol or 70 % ethanol was used. In order to purify pAdeno-X plasmid DNA employed in a mini-scale recombination, the following buffers 1 to 4 were prepared.

Buffer 1: 25 mM Tris-HCl (pH 8.0) including both of 10 mM EDTA and 50 mM glucose (after autoclaved, stored at 4 °C)
Buffer 2: 0.2M NaOH including 1 % of SDS (prepared immediately before the time of use, tightly sealed and stored at room temperature)
Buffer 3: 5 M KOAc (after autoclaved, stored at 4 °C)
Buffer 4: 10 mM Tris-HCl (pH 8.0) including both of ImM EDTA and 20 μg/ml of RNase (RNase is added immediately before use, stored at -20 °C)

(2) Purification of adenovirus and reagents for β-gal assay

HEK293 cell (ATCC #CRL1573) transformed by human type V adenovirus was used. HEK293 cell was cultured in a complete medium. The composition of the complete medium was DMEM (Dulbecco's Modified Eagle's Medium, the basal medium) supplemented with 100 unit/mL of sodium penicillin G, 100 μg/mL of streptomycin, 4 mM glutamine, and 10 % FBS. Sodium penicillin G solution was prepared at the concentration of 10,000 units/mL, and streptomycin sulfate solution was prepared at that of 10,000 μg/mL. They were stored as the stock solutions.
In the culture, plates with 60 mm of diameter, plates with 100 mm the diameter, 6-well plate, T75 and T175 flasks were used.
Trypsin-EDTA (the product code CC-5012) was purchased from Takara Bio Inc. Phosphate buffered saline (PBS, without $Ca^{2+}$ and $Mg^{2+}$) and Dulbecco's phosphate buffered saline (DPBS, with $Ca^{2+}$ and $Mg^{2+}$) were prepared. Also, 0.33 % neutral red stain solution, and 0.4 % trypan blue stain solution were used.
In β-gal assay, X-Gal (5-bromo-4-chloro-3-indolyl-β-D-galactopyranoside (25 mg/mL)) in dimethylformamide (DMF) solution was stored at -20 °C in a light resistant container. Luminescent β-gal Detection Kit II (the product code 631712) was used.

(3) Preliminary test

(3-1) Construction of recombinant adenovirus including lacZ (pAdeno-X-lacZ)

After thawing, HEK293 cells were removed from the solution including DMSO, and were resuspended in 10 mL of the complete medium. Then whole amount of the solution was transferred into the culture plate having a diameter of 100 mm. After HEK293 cells adhered to the bottom surface in the plate, the culture medium was removed. Subsequently, the cells were washed once with sterile PBS, and then, 1 ml of trypsin-EDTA was added to treat them for about 2 minutes.
Next, 10 mL of the complete medium was added to stop the reaction of trypsin, and then the cells were mildly suspended. After viable count of the cells was conducted, $10^5$ cells were transferred into the plate having 100 mm diameter including 10 mL medium, and spread out evenly.

Both of pShuttle2-lacZ (a positive control vector included in Adeno-X Expression System 1) and Adeno-X Viral DNA (both of PI-Sce I and I-Ceu I digested) included in the kit were used. According to a protocol attached in the kit, the recombinant adenovirus including lacZ was constructed. The recombinant adenovirus was infected to the target cell, SHED, and the expression of β-garactosidase was assayed to confirm that the vector was constructed.

(3-2) Construction of the recombinant pShuttle2 plasmid

Prior to the construction of the recombinant pShuttle2 Vector (herein below, it is referred to as "rpShuttle2 Vector".); E. coli DH5α was transformed with both of pShuttle2 Vector and pShuttle2-lacZ Vector, included in the kit. Transformants were selected on LB agar plate including 50 μg/mL of kanamycin (herein below, it is referred to as "LB/Kan".). Bacterial cells obtained from a single colony were streaked on new LB/Kan to be incubated at 37 °C for overnight.

Next, hTERT, bmi-1, E6, and E7 were cloned into pShuttle2 according to the following procedures. A pShuttle2 vector was cleaved by using a restriction enzyme suitable for these genes.

Next, referring pShuttle2 Vector Information Packet (PT3416-5) attached to the kit, multi cloning sites matching insert DNA were decided. The plasmid treated with the restriction enzyme was treated by using alkaline phosphatase to be purified.

According to the conventional method, target DNA fragments were prepared to be purified. The vectors digested with the restriction enzyme and the gene fragments were ligated. By using the ligation product, DH5α cells (competent cells) were transformed. A portion of the competent cell was taken to be transformed by using a control vector, pShuttle2-lacZ Vector included in the kit, and used as a positive control.

The mixture including transformed E. coli was plated on the LB/Kan agar plate to select kanamycin resistant (Kanr) transformants (colonies). Five to 10 Kan resistant clones were selected, and they were plated in a small amount of the liquid medium to be amplified. After confirmation that these clones have rpShuttle2 Vector, they were incubated overnight. Then, by using a commercially available silica gel adsorption column, the constructed plasmid DNA was purified according to the conventional method.

The plasmid DNA was treated with the restriction enzyme to be subjected to 1 % agarose gel electrophoresis; thereby the target recombinant plasmid was identified. By sequencing, the direction of the inserted fragment and the inserted site were confirmed to identify the positive clone.

The recombinant pShuttle2 plasmid DNA (herein below, it is referred to as "rpShuttle2 plasmid DNA") was directly transfected into the target cell, and then it was subjected to western blot to check the target protein expression preliminary.

(3-3) Double digestion of rpShuttle2 plasmid DNA with PI-Sce I/I-Ceu I

From the rpShuttle2 plasmid DNA produced as mentioned above, an expression cassette of the inserted gene was taken out by using both of PI-Sce I and I-Ceu I. According to in vitro ligation method written in the protocol attached to the kit, the expression cassette taken out was integrated into Adeno-X Viral DNA. 30 μL of PI-Sce I/I-Ceu I double-digestion solution for the rpShuttle2 plasmid DNA was prepared. The reagents shown in the following table 1 were entered into 1.5 ml of the sterilized micro centrifuge tube and mixed.

[Table 1]

| Reagent and others | Tube 1 (μL) | Tube 2 (μL) (lacZ control) |
|---|---|---|
| sterilized water | 19.5 | 19.5 |
| 10 x double-digention solution | 3.0 | 3.0 |
| rpShuttle2 plasmid DNA (500 ng/μL) | 2.0 | - |
| pShuttle2-lac Z plasmid (500 ng/μL) | - | 2.0 |
| PI-Sce I (1 unit/μL) | 2.0 | 2.0 |
| I-Ceu I (5 unit/μL) | 0.5 | 0.5 |
| 10 x BSA | 3.0 | 3.0 |
| Total | 30.0 | 30.0 |

Next, after sufficiently mixing, the micro centrifuge tube was lightly centrifuged, and then incubated for 3 hours at 37 °C.

The double digested reaction mixture (5 µL) was subjected to 1 % agarose/EtBr gel electrophoresis together with 1 kb ladder (DNA size marker).

(3-4) Extraction by using phenol: chloroform: isoamyl alcohol

70 µL of 1 x TE Buffer (pH 8.0) and 100 µL of PCI solution were added into the remains of the double-digestion solution (25 µL) in the centrifuge tube, and the tube was mixed by using a vortex. Then, the tube was centrifuged by using a micro centrifuge at 4 °C with 14,000 rpm for 5 minutes. Then, the aqueous layer was transferred to 1.5 ml of a clean centrifuge tube. Hereto, 400 µL of 95 % ethanol, 25 µL of 10 M ammonium acetate, and 1 µL of glycogen (20 mg/mL) were added, and then mixed them sufficiently by using the vortex.

Next, the mixture was centrifuged at 4 °C with 14,000 rpm for 5 minutes. Then, the supernatant was removed by aspiration to obtain a pellet. 300 µL of 70 % ethanol was added onto the pellet, and then it was centrifuged for 2 minutes with 14,000 rpm. The supernatant was carefully aspirated to remove, the pellet was air dried about for 15 minutes at room temperature.

After the pellet was dried, it was dissolved in 10 µL of sterilized 1 x TE Buffer (pH 8.0), and the solution was stored at -20 °C.

(4) Construction of the recombinant Adeno-X plasmid DNA

(4-1) Subcloning of the expression cassette into Adeno-X virus genome

The reagents shown in the following table 2 were added into the 1.5 ml of the sterilized micro centrifuge tube in order. Then, they were mildly mixed and lightly centrifuged. After that, the mixture was incubated at 16 °C for overnight.

[Table 2]

| Reagent and others | Liquid measure (µL) |
| --- | --- |
| PI-Sce I/I-Ceu I digested pShuttle2 plasmid DNA | 2.0 |
| PI-Sce I/I-Ceu I digested pShuttle2-lac Z plasmid DNA | - |
| sterilized water | 3.0 |
| 10 x DNA Ligation Buffer | 1.0 |
| Adeno-X Viral DNA(250 ng/µL) | 3.0 |
| DNA Ligase(1 unit/µL) | 1.0 |
| Total | 10.0 |

Both 90 µL of 1 x TE Buffer (pH 8.0) and 100 µL of PCI solution were added to each sample, and then they were mildly mixed by using vortex. The mixture was centrifuged at 4 °C with 14,000 rpm for 5 minutes, and the aqueous layer was transferred to 1.5 mL of the clean micro centrifuge tube. Then, 400 µL of 95 % ethanol, 25 µL of 10 M ammonium acetate solution, and 1 µL of glycogen (20 mg/ml) were added to the tube, and then it was mildly mixed by using the vortex.

The mixture was subjected to the centrifugation at 4 °C for 5 minutes with 14,000 rpm, and the supernatant was removed by the aspiration to obtain the pellet. The following ethanol precipitation was conducted as the same as those of (3-4).

After the pellet was dried, it was dissolved in 15 µL of the sterile deionized water.

(4-2) Swa I digestion of the recombinant Adeno-X plasmid DNA

The digestion solution as shown in the following table 3 was prepared, and added into each sample in the centrifuge tube. Then, they were incubated for 2 hours at 25 °C.

[Table 3]

| Reagent and others | liquid measure (µL) |
| --- | --- |
| ligation product | 15 |

(continued)

| Reagent and others | liquid measure ($\mu$L) |
|---|---|
| 10 x Swa I Digestion Buffer | 2.0 |
| 10 x BSA | 2.0 |
| Swa I (10 units/$\mu$L) | 1.0 |
| Total | 20.0 |

The mixture of 80 $\mu$L of 1 x TE Buffer (pH 8.0) and 100 $\mu$L of PCI solution were added to each sample, and then they were mildly mixed by using the vortex. They were centrifuged with micro centrifuge tube at 4 °C for 5 min with 14,000 rpm. The following ethanol precipitation was conducted as the same as those of (3-4). The dissolved solution of the pellet was stored at -20 °C until use.

(4-3) Confirmation of the E. coli transformant by the recombinant Adeno-X plasmid DNA

The electroporation competent E. coli was transformed with the Swa I digested products obtained in (4-2) by using Supercharge EZ10 Electrocompetent Cell (the product code 636756).

The transformant mixture was plated on the agar plate, which is the mixture of LB medium and ampicillin (final conc. 100 $\mu$g/mL) (herein after, it is referred to as "LB/Amp agar plate".); and then they are incubated at 37 °C for overnight to select ampicillin resistant (Ampr) transformants. About $10^6$ of colonies were obtained. The obtained colonies were checked by using Adeno-X System PCR Screening Primer Set attached to the product. The bacterial cells obtained from the single colony were inoculated in 5 mL of fresh LB/Amp liquid medium, and incubated overnight. In next day, according to the mini-scale method as mentioned below, Adeno-X plasmid DNA was purified.

(4-4) Mini-scale preparation of the recombinant Adeno-X plasmid DNA

5 mL of log-phase culture medium was centrifuged with 14,000 rpm for 30 seconds to remove the supernatant. The obtained pellet was centrifuged with 10,000 rpm for 1 minute again, and then the supernatant was removed by using the micropipette.

Hereto, 150 $\mu$L of the buffer 1 was added and mildly pipetted to resuspend. Then, 150 $\mu$L of the buffer 2 was added into the cell suspension, and mildly inverted to mix and stood for 5 minutes on ice. 150 $\mu$L of the buffer 3 was added to the cooled cell suspension, and then it was inverted to mix again and stood for 5 minutes on ice.

The cell suspension was centrifuged at 4 °C with 14,000 rpm for 5 minutes, and the transparent supernatant was transferred into 1.5 ml of the clean centrifuge tube. 450 $\mu$L of PCI solution was added to the supernatant in the tube, and then the tube was inverted to mix. Then, the tube was centrifuged at 4 °C with 14,000 rpm for 5 minutes, and the aqueous layer was transferred to the clean 1.5 ml of the micro centrifuge tube.

The following ethanol precipitation was conducted as the same as those of (3-4). The dissolved solution of the pellet was stored at -20 °C until use. The rDNA of the interest was analyzed by using the restriction enzyme and identified by PCR as described below.

(5) Restriction site analysis of the obtained rAdeno-X plasmid DNA

Analysis was performed by using both of PI-Sce I and I-Ceu I. The reagents shown in the following table 4 was entered into 1.5 ml of the micro sterilized centrifuge tube. Then, 30 $\mu$L of PI-Sce I/I-Ceu I double digestion solution was added there, and sufficiently mixed; and then it was lightly centrifuged to collect the contents.

[Table 4]

| Reagent and others | Amounts ($\mu$L) |
|---|---|
| sterilized water | 19.5 |
| 10 x double-digention solution | 3.0 |
| rpAdeno-X DNA (500 ng/$\mu$L)(500 ng/$\mu$L) | 2.0 |
| pShuttle2-lac Z plasmid (500 ng/$\mu$L) | - |
| PI-Sce I (1 unit/$\mu$L) | 2.0 |

(continued)

| Reagent and others | Amounts ($\mu$L) |
|---|---|
| I-Ceu I (5 unit/$\mu$L) | 0.5 |
| 10 x BSA | 3.0 |
| total | 30.0 |

The solution was incubated at 37 °C for 3 hours to treat with the restriction enzymes. The reaction mixture after the treatment was subjected to 1% agarose /EtBr gel electrophoresis to obtain the culture medium.

(6) Production of the recombinant adenovirus

(6-1) Preparation of the rAdeno-X plasmid DNA for HEK293 cell transfection

The reagents shown in the following table 5 was placed into the 1.5 ml of the sterilized centrifuge tube to be mixed, and the tube was lightly centrifuged by using the micro centrifuge. Then, it was incubated at 37 °C for 2 hours to treat with the rAdeno-X plasmid DNA with Pac I restriction enzyme.

[Table 5]

| Reagent and others | liquid measure ($\mu$L) |
|---|---|
| sterilized water | 20 |
| pAdeno-X plasmid DNA (500 ng/$\mu$L) | 10 |
| 10 x Pac I Digestion Buffer | 4 |
| 10 x BSA | 4 |
| Pac I (10 units/$\mu$L) | 2 |
| total | 40 |

Both of 60 $\mu$L of 1 x TE Buffer (pH 8.0) and 100 $\mu$L of PCI solution were added to the tube, and then it was mildly mixed by using the vortex. Then, the tube was centrifuged by using the micro centrifuge at 4 °C for 5 minutes with 14,000 rpm. The aqueous layer was carefully transferred into 1.5 ml of the clean sterilized centrifuge tube.
The following ethanol precipitation was conducted as the same as those of (3-4). The dissolved solution of the pellet was stored at -20 °C until use.

(6-2) Transfection of Pac I digested Adeno-X plasmid DNA into HEK293 cell

Before 24 hours of the plasmid DNA transection, HEK 293 cells were plated on the culture plate having 60 mm of the diameter so as that the cell number was about 1 to $2\times10^6$ (about 100 cells/mm$^2$). Then, they were incubated at 37 °C in the presence of 5% $CO_2$.
10 $\mu$L of Pac I-digested Adeno-X plasmid DNA was transfected to each culture plate to introduce Adeno-X DNA into the HEK293 cell, according to a standard transfection method (CalPhos Mammalian Transfection Kit, the product code 631312). It was confirmed whether CPE (cytopathic effect) occurs or not from the next day of the transfection.
One week later, the cells adhered on the bottom surface or side wall surface of the culture plate was released by mild mixing. The obtained cell suspension was transferred into 15 mL of the sterilized centrifuge tube having a conical bottom, and the tube was centrifuged at room temperature for 5 minutes with 1,500 x g.
The obtained precipitate was suspended in 500 $\mu$L of the sterilized PBS. The solution was subjected to the freeze-thaw procedures for 3 times, which is frozen in dry ice/ethanol and thawed in the incubator with 37 °C, to obtain the lysate in which the cells were sufficiently lyzed. Next, the tube was lightly centrifuged to remove floating matters, and then the supernatant was transferred into another sterilized tube to use immediately. The lysate, which was not used immediately, was stored at -20 °C.
250 $\mu$L of the lysate was added onto the cultured cells in the plate having 60 mm of the diameter, and continued to culture. Note that the titer of the adenovirus was determined by using anti-Hexon antibody included in Adeno-X Rapid Titer Kit (the product code 631028), according to the instruction manual (PT3651-1) of the kit.

(6-3) Virus amplification for preparing the virus having high titer

Before 24 hours of the titration assay, HEK293 cell were plated in a T75 flask, and they were incubated at 37 °C in the presence of 5% $CO_2$ overnight to be confirmed that they became 50 to 70% of confluent.

Next day, the medium was exchanged to the fresh one including the virus to infect them with the virus at MOI = 10. After the incubation at 37 °C in the presence of 5% $CO_2$ for 90 minutes, the flask was taken out and 10 mL of the medium was added into the flask.

After 3 to 4 days culture at 37 °C in the presence of 5% $CO_2$, CPE was confirmed. At the timing that 50% of the cells were released, the released cell suspension was prepared as the same as that described above; and then the suspension was transferred to 15 mL of the sterilized centrifuged tube with the conical bottom. The freeze and thaw procedure as the same as that described above was performed, and the cells were lyzed. By using Adeno-X Rapid Titer Kit (the product code 631028), $10^7$ PFU/mL of titer was obtained.

Western blotting was performed to confirm whether the packaged adenovirus genome has copies of the specific transcription unit against the target gene in operative form.

(7) Adenovirus infection to the target cells

(7-1) Infection to the target cells

Before 24 hours of the infection, 1 x $10^6$ cells of SHED were plated on 6-well plate. Next day of the plating, the medium was removed, and 1.0 mL of the medium including the virus was added to the center of each plate. The medium was spread out evenly on a monolayer formed by the SHED.

The plates were incubated at 37 °C for 4 hours in the presence of 5% $CO_2$ to establish the virus infection to SHED. Next, the fresh medium was added to the plates, and then they were incubated at 37 °C in the presence of 5% $CO_2$. From 24 to 48 hours after the infection, the expressions of the introduced genes were analyzed time dependently.

(7-2) Analysis of the β-galactosidase expression of the infected cells

The expression of β-galactosidase in the adherent cells infected with the Adeno-X-lacZ was assayed by using Luminescent β-gal Detection Kit II (the product code 631712, Clontec Laboratories Inc.).

(Example 2) Manufacturing of SHED

(1) Isolation of dens deciduous stem cell

Exfoliated dens deciduous obtained from 10 years old healthy boy were used. After the exfoliated dens deciduous was disinfected with Isodine solution, a crown of the teeth was horizontally cut by using the dental diamond point, and then the dental pulp tissue was collected by using the dental reamer. The obtained dental pulp tissue was digested in the solution including 3 mg/mL of type I collagenase and 4 mg/mL of disperse at 37 °C for 1 hour. Next, the solution was filtrated by using 70 mm of cell strainer (Falcon).

The filtrated cells were resuspended in 4 mL of the medium to be plated into the culture dish for adherent cell having the 6 cm in diameter. DMEM (Dulbecco's Modified Eagle's Medium) including 10 % FCS was added into the dish and cultured for about 2 weeks in the incubator (desk top cell culture device for personal use, 9000EX series, Wakenbiotech Co., LTD.) adjusted as 5% $CO_2$, at 37 °C. The adherent cells formed colonies (the dental pulp stem cells) were treated by using 0.05% trypsin/EDTA for 5 minutes at 37 °C, and then the cells released from the dish were collected.

Next, the adherent cells selected as mentioned above were plated on the culture dish for the adherent cells (a collagen coat dish), and they were incubated as primary culture in the incubator adjusted as 5% $CO_2$ at 37 °C to obtain the primary cultured cell. When the cells became macroscopically sub-confluent (about 70 % of the surface of the culture container was covered by the cells), or confluent, the cells were treated by using 0.05% trypsin/ EDTA at 37 °C for 5 minutes to be released from the container, and then collected.

Thus obtained cells were again plated on the dish including the medium, and perform passage in several times to be grown up to about 1 x $10^7$ cells/mL. The obtained cells were stored in the liquid nitrogen.

After that, by using the primary cultured cells, the passage was performed with the medium at the cell concentration of 1 x $10^4$ cells/cm$^2$. In the experiment, the cells passed from 1 to 3 times were used. The human BMMSC (the bone marrow mesenchymal stem cell, Bone Marrow Mesenchymal stem cells) was purchased from Lonza Group Ltd. and cultured according to the manufacturer's instruction manual.

As described above, the human exfoliated dens deciduous dental pulp stem cells (SHED) were obtained. Among the obtained SHED, about 1 x $10^6$ cells of STRO-1 positive cells were sorted as follows from each sample by using FACSTARPLUS (Becton, Dickinson and Company).

According to the manufacturer's instruction manual of the bromodeoxyuridine BrdU staining kit (Invitrogen), BrdU was incorporated into the cells for 12 hours to evaluate the growth rate of SHED (n = 3 in each group). The experiments were repeated for 5 times. After one-way analysis of variance, Tukey-Kramer test was performed to evaluate statistical significant difference.

In order to detect STRO-1 with immunofluorescence, SHED was fixed with 3% paraformaldehyde, rinsed twice with PBS and then treated with 100 mM of glycine for 20 minutes. Next, these cells were permeabilized with 0.2% Triton-X (Sigma-Aldrich) for 30 minutes. Then, they were incubated in the mixture of 5 % donkey serum and 0.5% bovine serum albumin for 20 minutes.

Next, the cells were incubated with the primary antibody, mouse antihuman STRO-1 antibody (1:100, R&D Inc.) for 1 hour, then incubated with the secondary antibody, goat anti-mouse immunoglobulin M-FITC antibody (1:500, Southern Biotech Corp.) for 30 minutes, and then mounted by using Vector Shield DAPI (Vector Laboratories Inc.).

After that, $\alpha$-MEM supplemented with 15 % FBS was added to the 6 well plate, and then the sorted cells were plated in each well for preparing clones. About 300 colonies among the proliferated cells were pooled for the test.

(2) Transgenesis

As described above, 4 genes, bmi-1, E6, E7 and hTERT were integrated into the adenovirus vector to manufacture a virus vector to express the gene products. As a reference, the control vector not integrated the genes was manufactured.

SHED was plated on the collagen coat dish having 100 mm$\phi$ of the diameter at the concentration of 1 x 10$^6$ cells, and then DMEM supplemented with 10% FBS was added. They were cultured until sub-confluent. The medium was removed by aspiration, and 500 $\mu$L of the virus solution diluted with the medium was added (MOI = 10), and then incubated at 37 °C for 1 hour in the 5% $CO_2$ incubator for the virus vector infection. After 48 hours from the infection, the infected cells were incubated for 10 days in the medium supplemented with puromycin (1 pg/mL) to select. Then 500 to 600 of the resistant clones were pooled. Every 3 to 4 days, about 0.5 x 10$^5$ cells of SHED was plated to the culture dish having 100 mm$\phi$ of the diameter to perform passage. SHED to which the genes were transferred was named SHED-T, and SHED to which the genes were not transferred was named SHED-C.

(Example 3) Study of feature of SHED

(1) Measurement of the growth rates of SHED-C and SHED-T

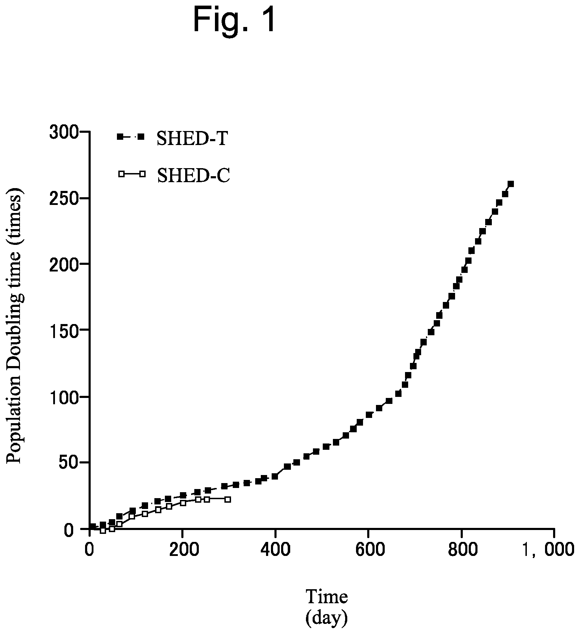

Status of the population doubling time of SHED-T (the gene transferred SHED) was shown in Fig. 1. In the figure, a vertical axis shows the population doubling time number (cell division number, times), and an abscissa axis shows the time period (date of culture). The status that SHED in culture did not divide for 1 month was as an evaluation standard of the aging.

The proliferation of SHED-C has stopped at about 30 times to enter aging or proliferation termination phase. In contrast, SHED-T passed over 250PD and proliferated after 800 days have passed.

(2) Flow cytometry analysis

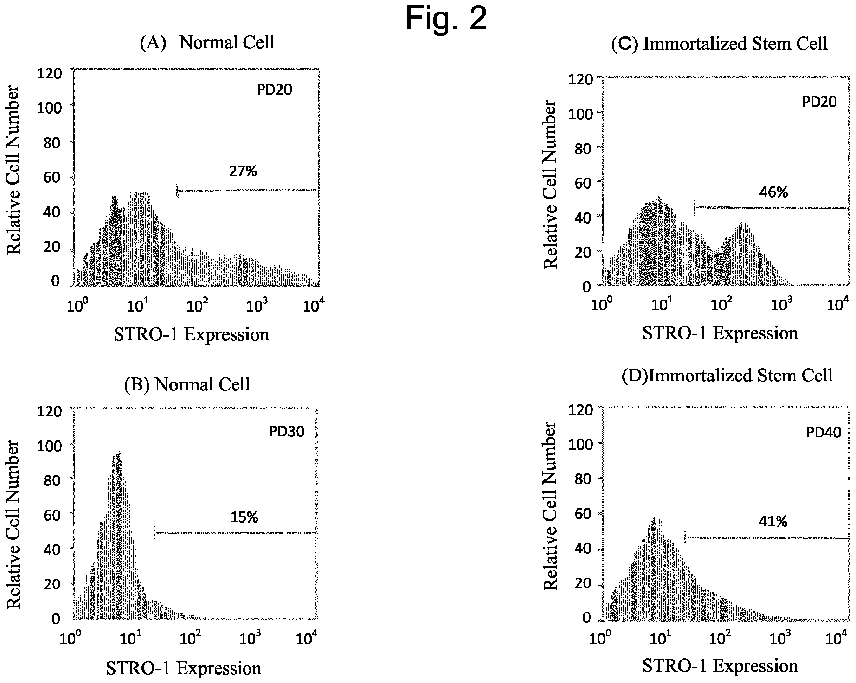

In order to obtain a suspension containing a single cell, the adherent monolayer cells were digested with trypsin/EDTA. The anti-STRO-1 monoclonal antibody (1:100) was added to 2 x 10$^5$ cells and stood to analyze by using FACS Calibur flow cytometer (Becton, Dickinson and company). When the fluorescence level of them was higher at the rate of more than 99 % compared to the reference antibody having the same isotype as these corresponding to test antibody, the expression was evaluated to be positive. In both of SHED-T and SHED-C, the primary and later passage cells were fixed, and stained with FITC binding STRO-1 antibody. Then, they were analyzed by using the flow cytometry. Every test was conducted twice. In SHED-C, the ratio of the STRO-1 positive cells was 27% at PD20, and decreased to 15% at PD30 (Fig. 2(A) and (B)). The ratio of the STRO-1 positive cells in SHED-T was 46% at PD20 and 41% at PD40, respectively (Figs. 2(C) and (D)).

(3) Study for the differentiation ability

The differentiation abilities of SHED-C and SHED-T at PD0, PD10 and PD20 were studied by the forming ability of the newly generated bone mass and histological stain of the tissue.

Firstly, 2.0 x 10$^6$ cells of SHED-C or SHED-T were mixed with 40 mg of ceramic powder of hydroxyapatite/tricalcium phosphate (HA/TCP) (Olympus Corporation), and then the mixture was inoculated subcutaneously under a dorsal surface of immunocompromised mouse at 10 weeks old (NIH-bgnu-xid, female, Harlan Sprague Dawley Inc.).

Eight weeks after the inoculation, the inoculant was recovered, and fixed with 4% formalin to decalcify. Then, it was buffered by using PBS solution including 10% EDTA for paraffin embedding. A part of it was stored in 70% ethanol for embedding in resin.

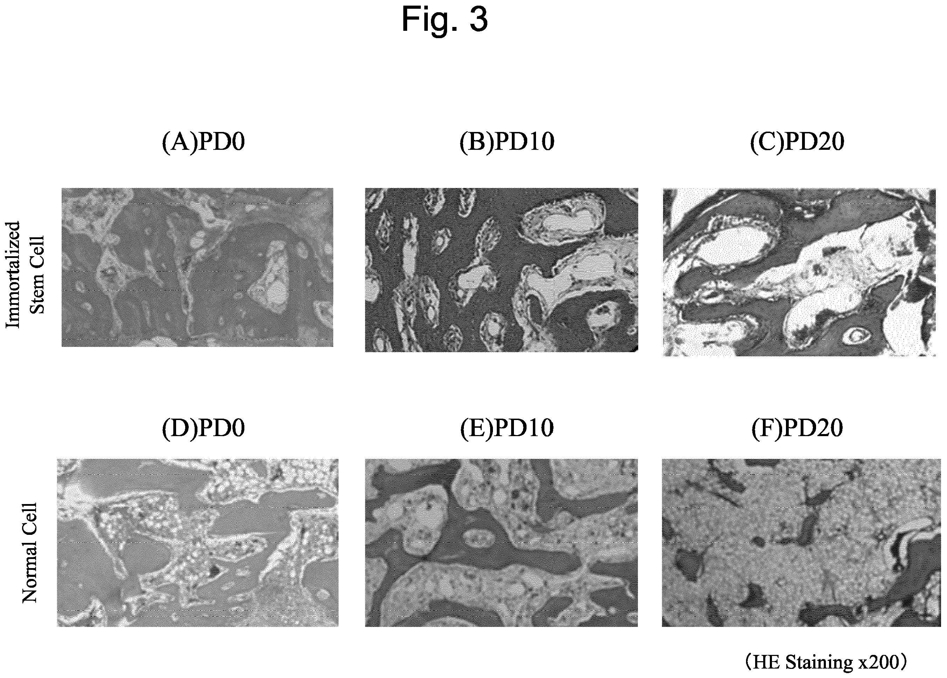

A paraffin section was deparaffinized, and hydrated. After that, the section was stained with hematoxylin and eosin (herein below, it is referred to as "H&E".). Figs. 3(A) to (C) show the stained images of SHED-T (the immortalized stem cell) at PD0 to PD20, and Figs. 3(D) to (F) show the stained images of SHED-C (the normal cell) at PD0 to PD20. In order to quantify of the new born formation in vivo, the specified positions were chosen, and the area of the new born and the sight area were calculated to obtain the newly generated born mass from these values for the inoculant formed after SHED-T inoculation or SHED-C inoculation respectively.

$$\text{Newly generated born mass} = \text{Newly generated born area/sight area} \times 100$$

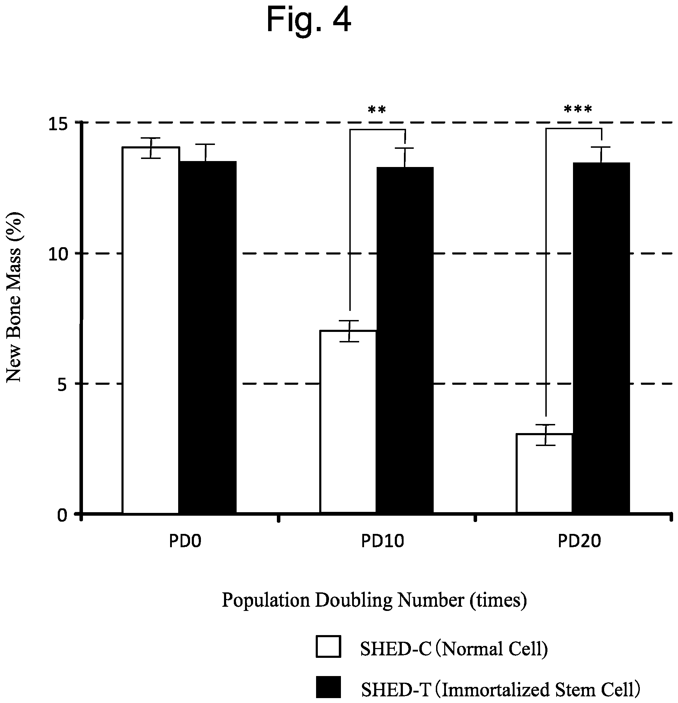

Fig. 4 shows the change of the newly generated born mass of SHED-T and SHED-C at each population doubling number (doubling time). In the figure, ** shows $p < 0.05$, *** shows $p < 0.01$. Note that the newly generated born mass was obtained by using the equation.

As shown in Fig. 4, the newly generated bone mass was decreased depending on the increase of the population doubling time in SHED-C, and it was decreased to about 1/5 at PD20 compared to that of PD0. In contrast, the bone generated bone mass was not changed till PD20 in SHED-T, and the bone mass in SHED-T showed 5 times higher than that of SHED-C at PD20.

(4) Evaluation of canceration activity

$1 \times 10^6$ cells of SHED-C cells or SHED-T cells were inoculated to the subcutaneous tissue of the immune compromised mice. After inoculation, we observed more than 30 days. However, the tumor was not formed during the observation term in any mice to which the cells were inoculated. Also, all of the clones from the cultured cells between 40 to 200PD did not show any morphological change in SHED-T cells.

From the above, it was demonstrated that SHED-T had no canceration activity.

(5) Evaluation

It was demonstrated that SHED-T had proliferation ability, holding differentiation ability even after 260PD. However, SHED-C had the differentiation ability, but aged not more than 30PD.

As described above, it was demonstrated that SHED-T became the immortalized stem cell, and was suitable for large scale production of SHED supernatant having higher activity.

(Example 4) Preparation of condition medium

The immortalized SHED prepared in the example 1 was cultured in serum free medium for 48 hours under the following conditions of oxygen concentration of 20%, 10%, 5% or 1%. Then, the supernatants were collected.

The production amount of cytokines shown in the following Table 6 was measured by using ELISA kit (R & D systems, Inc., Human IGF-1 Quantikine kit (Catalogue No.: DG100), Human T GF-β Quantikine Elisa kit (Catalogue No.: DB100B), and Human VEGF Quantikine Elisa kit (Catalogue No.: DVE00).

[Table 6]

| Group | Oxygen co ncentration (%) | Production amount of cytokine (pg/mL) | | | |
|---|---|---|---|---|---|
| | | IGF-1 | VEGF | TGF-β1 | SDF-1 |
| I | 20 | 1,400 | 500 | 350 | 15 |
| II | 10 | 1,800 | 550 | 500 | 17 |
| III | 5 | 1,900 | 600 | 600 | 30 |
| IV | 1 | 2,100 | 800 | 1,770 | 45 |

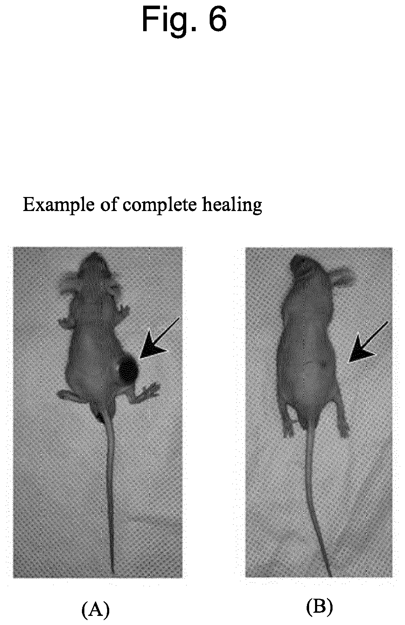

As shown in Fig. 6, the production amount of IGF-1, VEGF, TGF-$\beta$ and SDF-1 were significantly higher under the condition of low oxygen concentration. It shows that the production amounts of at least four cytokines described above were significantly increased by culturing under the condition of low oxygen concentration.

(Example 5) Study of therapeutic effect by using tumor bearing animal

Mouse squamous cell cancer strain SCCVII (provided from Dr. Nishimura at the medical school of Kinki University) was cultured in DMEM supplemented with 10% of FBS (Gibco Co.) at 37 °C for 1 week. Then, the cells were dispersed in each cell by using PBS buffer including 0.5% trypsin. The viable cells were counted by using trypan blue staining. Then, $1 \times 10^6$ cell/mL of the suspension was prepared with 1 mL of PBS buffer.

0.5 ml of the prepared cell suspension ($5 \times 10^5$ cells/mouse) was subcutaneously injected by using 18G injection needle (product by Terumo Co.) to dorsal of 50 mice (C3H/He, 7 week-old, purchased from Chubu Kagaku Shizai Co., LTD.). Each group had 10 mice.

1 mL of buffer only was injected through the mouse vein for mice of the control group; and 1 mL of the culture supernatant was injected through the mouse vein for the mice of the group I to the group IV (hereinafter, it is sometime referred to as "GI to GIV") respectively.

After the cancer cells were injected to them, each of them was kept for group under the same conditions as described above.

The mice in the group was put in one cage to be kept under the condition of light and dark for 12 hours at 25 $\pm$ 0.5 °C with 50% humidity. Water and feed were taken freely. Diameters of the tumors in the mice were measured by using calipers every day.

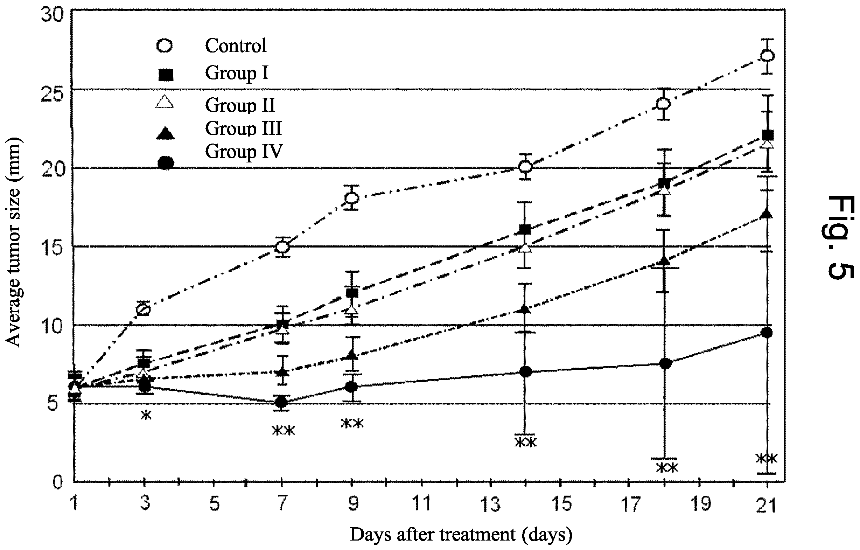

When the diameters of the tumors, formed by SCCVII injection in the mice, were over 6 mm, 1 mL of the culture supernatant, obtained under the varied oxygen conditions, was injected to each mouse once through the tail vein of each mice of GI to GIV. The increased tumor diameters of each mouse in each group were shown in Fig. 5.

In GI, the tumor diameter exceeded 10 mm on the 3rd day from starting of the injection, and 20 mm on 14th day. The tumor diameter was exceeded 25 mm on 21st day. Both of GII and GIII group showed significantly slow growth rate of the tumor diameter compared to that of GI (*shows $p<0.05$). The diameter exceeded 10 mm on the 7th day from the start of the injection, and it exceeded 20 mm on the 9th day.

In contrast, GIV showed the significantly slow growth rate (**shows $p<0.01$), and the average of the tumor diameters of the GIV group mice did not exceed 10 mm even on 21st day from the start of the injection. However, the standard deviation was increased after 14th day, because some mice in GIV showed tumor regression. Fig. 6 shows the pictures of the mouse, (A) shows the maximal size tumor diameter, and (B) shows complete disappearance of the tumor.

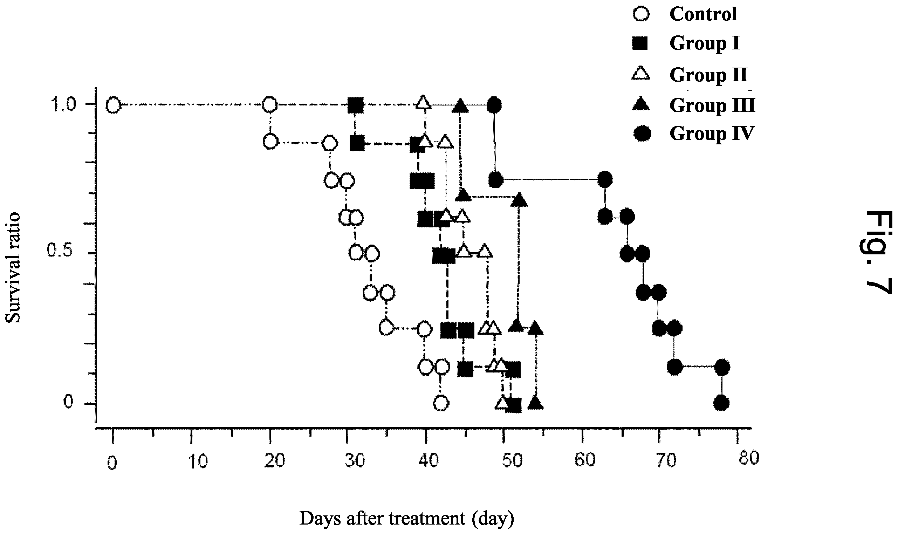

Fig. 7 shows survival ratio the mice in each group. The differences of the growth rate of tumor diameters were reflected in the mice survival ratio. All mice in GI were dead on 41st day from the start. Also, all mice in GII or GIII were dead at 50th day from the start. In contrast, about 80% of mice in GIV were survived even after 60 days from the start. All the mice in GIV died on 78th day from the start. The lifetime of the mice in GIV were about twice as long compared to that of GI

(Example 6) Study related to the change of tumor and surrounding tissue thereof

The behaviors of macrophages to tumor tissue were observed by using in vivo imaging. 5 ml of thioglycolate solution (2% of Brewer's thioglycolate medium (Difco Co.) was injected into mouse i.p. 4 days later, the peritoneal lavage of the mice was conducted with PBS, and it gave $10^7$ cells of macrophage. Among obtained macrophages, $3 \times 10^6$ macrophage cells were mixed with 5 $\mu$g/mL of pigment (MolecularTracer Dir (Summit Pharmaceutical International Co.)) and 0.5% of ethanol to be labelled.

One mL/mouse of the culture supernatant prepared in the example 2 was injected into the mice in each group through their tail veins in single dose. The amounts of cytokines in the culture supernatant were measured by using Human IGF-1 Quantikine Elisa kit, Human TGF-$\beta$ Quantikine Elisa kit, Human VEGF Quantikine Elisa kit as described above. The behaviors of the labeled macrophages were observed by using Xenogen IVIS 200 series system (Xenogen, Alameda, CA). The imaging was conducted by using the recommended IVIS filter set (excitation of 710 nm/fluorescence of 760 nm), and it was performed at 748 nm of excitation wavelength and 780 nm of fluorescence wavelength.

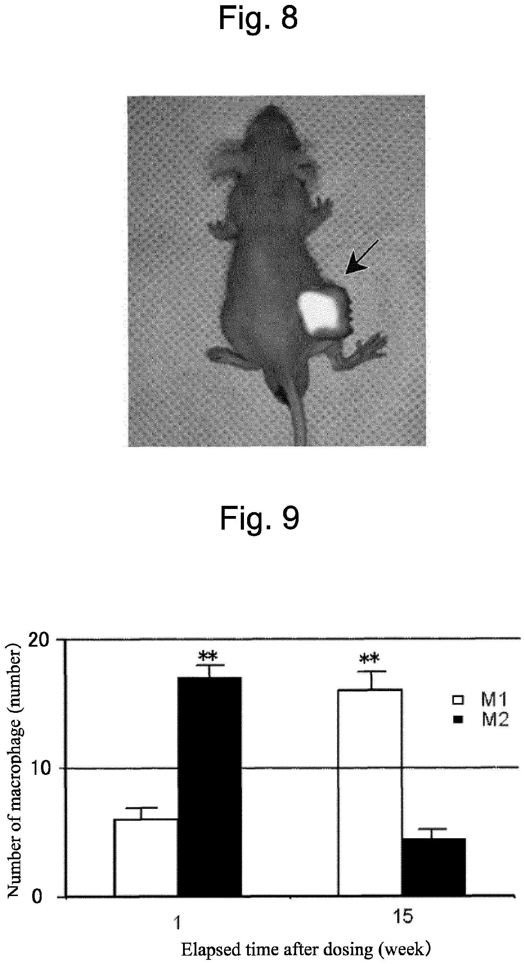

The labeled macrophages in GIV began to migrate within 1 hour, and they accumulated so as to surround the entire tumor (Fig. 8). In the figure, invasive image of the macrophage was observed at the base of right hind leg on which the tumor was formed. Such accumulation of the macrophage around the periphery of the tumor was not observed in the mice of GI to GIII.

(Example 7) Histological study

5x10$^5$ cells of SCCVII were injected into CH3/He mouse. The formed tumors were excised including their periphery tissues on 1 week later and also 15 week later for histological autopsy. In the treatment group (GIV), there was an example, wherein the tumor necrosis was observed on 15 week from the start (Fig. 6).

Also, CDllb antibody (BioLegend Inc.) as macrophage maker, CD206 antibody (Abcam plc) as M2 macrophage maker, and ED1 (CD68) antibody (Millipore Corp.) as M1 macrophage maker were employed respectively for staining the macrophages. Then, the ratio of M1 to M2, which was subpopulation of macrophage, was studied. The result was shown in Fig.9.

As shown in Fig.9, the ratio of M2 was larger (M2 dominant) at the early stage of tumor generation (1 week after cell injection). In contrast, the ratio of M1 and M2 was reversed (M1 dominant) at the later stage (15 week after the cell injection). The investigation by using Western blotting method resulted that M2 mainly expressed TGF-β superfamily; however, M1 expressed TGF-β inhibitors (Trabedersen, LAP 12009) and the like.

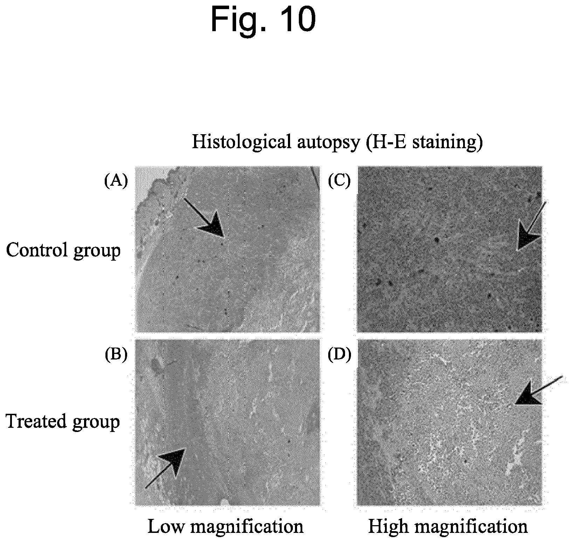

Fig. 10 shows the stained images of the tumor tissue of the mice, which were obtained from the mice in the control group or those in treated group stained with eosin-hematoxylin. Comparison between the stained images of the control group (A) and treated group (B) with low magnification gave the result that there are some dead area wherein the tumor cells were died in the treatment group image. In high magnification, the difference between these groups was clearly observed (Fig. 10 (C) and (D)).

From the above, it was shown that the dental pulp stem cell responses to the hypoxic condition to enhance the specific cytokine related to the tumor growth and activate them. As a result, they give promoted migration ability to the macrophages, and keep macrophage highly gathered surrounding to the tumor tissue.

Furthermore, it was considered that the macrophage gathered around the tumor destroy the tumor tissue by using original phagocytosis ability thereof and at the same time inhibit tumor growth through the TGF-β.

**Industrial Applicability**

The present invention is useful to the pharmaceutical field.

**Claims**

1. A pharmaceutical composition for cancer therapy prepared by a method comprising the steps of:

   manufacturing immortalized stem cells of dens deciduous dental pulp stem cells derived from mammal dental pulp by transfection; and
   preparing a conditioned medium by culturing said immortalized stem cells in a serum free medium for a predetermined period under the condition of hypoxic concentration of 0.5% or more but 10% or less of oxygen concentration between the temperature of 23 to 27 °C.

2. The pharmaceutical composition for cancer therapy according to the claim 1, wherein said predetermined period is 40 to 56 hours.

3. The pharmaceutical composition for cancer therapy according to the claim 1 or 2, wherein said hypoxic concentration means that oxygen concentration is 5% or less of.

4. The pharmaceutical composition for cancer therapy according to the claim 3, wherein said hypoxic concentration means the oxygen concentration is 1% or less.

5. The pharmaceutical composition for cancer therapy according to the claim 4, wherein the immortalized stem cells are manufactured by transfecting four genes selected from the group consisting hTERT, bmi-1, E6, E7, Oct3/4, Sox2, Klf4, c-Myc, and p16INK4a into the dens deciduous dental pulp stem cells.

6. A pharmaceutical preparation for cancer therapy comprising the pharmaceutical composition according to any of claims 1 to 5 as active ingredient.

7. The pharmaceutical preparation for cancer therapy according to claim 6, wherein said cancer is solid tumor.

8. A pharmaceutical composition as defined in any one of claims 1 to 5 for use in treating cancer.

**9.** A pharmaceutical preparation as defined in claim 6 or 7 for use in treating cancer.

**Patentansprüche**

**1.** Pharmazeutische Zusammensetzung für die Krebstherapie, hergestellt durch ein Verfahren, das die folgenden Schritte umfasst

Herstellung immortalisierter Stammzellen aus dentalen Milchzahn-Pulpa-Stammzellen, die aus der Zahnpulpa von Säugetieren durch Transfektion gewonnen wurden; und
Herstellung eines konditionierten Mediums durch Kultivieren der immortalisierten Stammzellen in einem serumfreien Medium für einen vorbestimmten Zeitraum unter der Bedingung einer hypoxischen Konzentration von 0,5% oder mehr, aber 10% oder weniger der Sauerstoffkonzentration zwischen der Temperatur von 23 bis 27°C.

**2.** Pharmazeutische Zusammensetzung für die Krebstherapie nach Anspruch 1, wobei der vorbestimmte Zeitraum 40 bis 56 Stunden beträgt.

**3.** Pharmazeutische Zusammensetzung für die Krebstherapie nach Anspruch 1 oder 2, wobei die hypoxische Konzentration bedeutet, dass die Sauerstoffkonzentration 5% oder weniger beträgt.

**4.** Pharmazeutische Zusammensetzung für die Krebstherapie nach Anspruch 3, wobei die hypoxische Konzentration bedeutet, dass die Sauerstoffkonzentration 1% oder weniger beträgt.

**5.** Pharmazeutische Zusammensetzung für die Krebstherapie nach Anspruch 4, wobei die immortalisierten Stammzellen durch Transfektion von vier Genen, ausgewählt aus der Gruppe bestehend aus hTERT, bmi-1, E6, E7, Oct3/4, Sox2, Klf4, c-Myc und p16INK4a, in die dentalen Milchzahn-Pulpa-Stammzellen hergestellt werden.

**6.** Pharmazeutische Zusammensetzung für die Krebstherapie, umfassend die pharmazeutische Zusammensetzung nach einem der Ansprüche 1 bis 5 als aktiven Bestandteil.

**7.** Pharmazeutische Zubereitung für die Krebstherapie nach Anspruch 6, wobei der Krebs ein solider Tumor ist.

**8.** Pharmazeutische Zusammensetzung wie in einem der Ansprüche 1 bis 5 definiert zur Verwendung bei der Behandlung von Krebs.

**9.** Pharmazeutische Zubereitung wie in Anspruch 6 oder 7 definiert zur Verwendung bei der Behandlung von Krebs.

**Revendications**

**1.** Composition pharmaceutique pour thérapie anticancéreuse préparée par un procédé comprenant les étapes de :

fabrication de cellules souches immortalisées à partir de cellules souches de pulpe dentaire de dents de lait dérivées de pulpe dentaire de mammifère par transfection ; et
préparation d'un milieu conditionné par une mise en culture desdites cellules souches immortalisées dans un milieu sans sérum pendant une durée prédéterminée sous la condition d'une concentration hypoxique de 0,5 % ou plus mais 10 % ou moins de concentration d'oxygène entre la température de 23 à 27°C.

**2.** Composition pharmaceutique pour thérapie anticancéreuse selon la revendication 1, dans laquelle ladite période prédéterminée est de 40 à 56 heures.

**3.** Composition pharmaceutique pour thérapie anticancéreuse selon la revendication 1 ou 2, dans laquelle ladite concentration hypoxique signifie que la concentration en oxygène est de 5 % ou moins.

**4.** Composition pharmaceutique pour thérapie anticancéreuse selon la revendication 3, dans laquelle ladite concentration hypoxique signifie que la concentration en oxygène est de 1 % ou moins.

**5.** Composition pharmaceutique pour thérapie anticancéreuse selon la revendication 4, dans laquelle les cellules souches immortalisées sont fabriquées par transfection de quatre gènes choisis dans le groupe comprenant hTERT, bmi-1, E6, E7, Oct3/4, Sox2, Klf4, c-Myc et p16INK4a dans les cellules souches de pulpe dentaire de dents de lait.

**6.** Préparation pharmaceutique pour thérapie anticancéreuse comprenant la composition pharmaceutique selon l'une quelconque des revendications 1 à 5 en tant qu'ingrédient actif.

**7.** Préparation pharmaceutique pour thérapie anticancéreuse selon la revendication 6, dans laquelle ledit cancer est une tumeur solide.

**8.** Composition pharmaceutique telle que définie dans l'une quelconque des revendications 1 à 5 à utiliser pour traiter un cancer.

**9.** Préparation pharmaceutique telle que définie dans la revendication 6 ou 7 à utiliser pour traiter un cancer.

# Fig. 1

# Fig. 2

(A)  Normal Cell

(C) Immortalized Stem Cell

(B) Normal Cell

(D)Immortalized Stem Cell

EP 3 093 023 B1

# Fig. 3

(A)PD0        (B)PD10        (C)PD20

Immortalized Stem Cell

(D)PD0        (E)PD10        (F)PD20

Normal Cell

(HE Staining x200)

EP 3 093 023 B1

# Fig. 4

# Fig. 5

# Fig. 6

Example of complete healing

(A)                              (B)

# Fig. 7

# Fig. 8

# Fig. 9

# Fig. 10

Histological autopsy (H-E staining)

(A) Control group — Low magnification
(B) Treated group — Low magnification
(C) — High magnification
(D) — High magnification

Low magnification       High magnification

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011118795 A
- WO 2013147082 A
- EP 2307539 A

**Non-patent literature cited in the description**

- **YUE WANG et al.** *PLOS ONE,* January 2013, vol. 8 (1), e54296
- **M. CELESTE SIMON ; BRIAN KEITH.** *Nature Reviews/ Molecular Cell Biology,* April 2008, vol. 9, 285-296
- **ARANHA A M F et al.** *Journal of endodontics,* October 2010, vol. 36 (10), 1633-1637